(19)

**Europäisches Patentamt**

**European Patent Office**

**Office européen des brevets**

(11)  **EP 4 517 774 A1**

(12)  **EUROPEAN PATENT APPLICATION**

(43) Date of publication:
**05.03.2025 Bulletin 2025/10**

(21) Application number: **23194855.5**

(22) Date of filing: **01.09.2023**

(51) International Patent Classification (IPC):
***G16H 50/20*** (2018.01)

(52) Cooperative Patent Classification (CPC):
**G16H 50/20; G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
- **VAN GORP, Hans
Eindhoven (NL)**

- **DOS SANTOS DA FONSECA, Pedro Miguel
Ferreira
Eindhoven (NL)**
- **VAN GILST, Merel Marietje
5656AG Eindhoven (NL)**
- **OVEREEM, Sebastiaan
Eindhoven (NL)**
- **VAN SLOUN, Ruud Johannes Gerardus
Eindhoven (NL)**

(74) Representative: **Philips Intellectual Property &
Standards
High Tech Campus 52
5656 AG Eindhoven (NL)**

(54)  **SYSTEM AND METHOD FOR GENERATING OUTPUT DATA USING A GENERATIVE MODEL**

(57)     A mechanism for generating output data using a generative model. The generative model comprises a plurality of score-based neural networks, each configured to generate an initial score processable, using a sampling technique, to produce an instance of example data. The initial scores are combined to define a combined score. The combined score is processed using the sampling technique to generate the output data. Each score-based neural network is trained using a different set of training data.

FIG. 2

**Description**

BACKGROUND OF THE INVENTION

There is an increasing interest in the development and use of generative models to generate output data from some input data. There are a wide variety of uses for generative models, such as those designed for performing one or more medical or clinical tasks (such as sleep analysis, image reconstructions, image segmentation and so on).

In score-based generative models, the input data is typically processed to produce a score, sometimes labelled a score function, that defines one or more characteristics of a probability distribution for the output data. The score is then processed using an appropriate sampling technique to generate or predict the output data. In particular, the probability distribution defined by the score is sampled using the sampling technique to generate some output data. The input data and the output data may, for instance, be multi-dimensional.

There is an ongoing desire to improve the flexibility of use and accuracy of generative models.

SUMMARY OF THE INVENTION

The invention is defined by the claims.

According to examples in accordance with an aspect of the invention, there is provided a processing system configured to: receive a plurality of initial scores, wherein each initial score is an output of a different score-based neural network of a generative model and defines a probability distribution for sampling output data, wherein each score-based neural network has been independently trained using a different set of training data; process the initial scores to generate a combined score; and process the combined score, using a sampling technique, to produce output data of the generative model.

The present disclosure proposes an approach for generating a combined score from a plurality of initial scores. In the context of the present disclosure, the term score takes on the meaning used in the field of generative models, e.g., to comprise information that represents the distribution of probable values for an output. Each initial score is generated by a different score-based neural network, e.g., that processes a respective set of input data to produce the initial score. Each set of input data pertains or relates to a same entity, e.g., a same subject or individual. The set of input data processed by each score-based neural network may be the same set of input data or, preferably, different sets of input data. For instance, each set of input data may contain data for a different set or grouping of characteristics of the entity (e.g., subject or individual).

By combining scores from independent score-based neural networks, each trained on a different input type, a flexibility in the input types used is achieved. The inventors have recognized that this provides an output that is at least comparable, in terms of quality, to a generative model employing a score-based neural network simultaneously trained on all input types.

This allows input types for the generative model to be added or removed without requiring the entire generative model to be retrained. Rather, in the case of adding an input type, only an (i.e., one) additional score-based neural network independently trained on the new input type is required, while in the case of removing an input type, the score-based neural network trained for the removed input type is simply not used.

Each initial score may be a vector differential operator.

In some examples, each set of training data comprises training data for a respective one of a plurality of different data types; and the processing system is further configured to: receive a plurality of sets of input data, each set of input data containing input data for a respective one of the plurality of different data types; and provide each set of input data to a score-based neural network trained using a set of training data of the same type to generate the plurality of initial scores.

The generative model may be a clinical assessment tool. In other words, the generative model may be configured to generate output data for aiding a clinical decision, such as aiding in the understanding, analysis and/or assessment of the condition of a medical subject such as a patient. Examples of clinical assessment tools include those designed for aiding in the performance of any suitable medical task. Example clinical assessment tools include a model for determining a hypnogram of a sleep session by processing physiological signals; predicting whether or not one or more pathologies (e.g., cardiac defects, respiratory problems etc.) are present by processing physiological signals; identifying one or more target anatomical elements (e.g., tumors, growths, cancers etc.) in one or more medical images; segmenting anatomical elements in one or more medical images; denoising of medical images; predictive generation of medical image data from a portion of medical image data; and so on. This list of examples is non-exhaustive, and the skilled person would be readily capable of identifying additional functions and/or tasks to be performed by a generative model acting as a clinical assessment tool.

The output data of the generative model may be a time series, i.e., a sequence or vector of data values that represent different values of output data over time.

The sets of training data together comprise physiological signals for one or more subjects during one or more sleep sessions. In other words, the generative model may be trained to analyze a subject's sleep.

The physiological signals may comprise one or more of: an electroencephalography signal, an electrooculography signal, an electromyography signal, an electrocardiography signal, a ballistocardiography signal, a seismocardiography signal, a pulse oximetry signal, a photoplethysmographic signal, a snoring microphone audio signal, an ambient microphone audio signal, body movement signal, a respiratory movement signal, a respiratory effort signal and/or a respiratory airflow signal.

In some examples, the time series is a hypnogram or hypodensity graph (e.g., a sleep stage probability over time). A hypnogram is a time series of estimated sleep stages for a subject during a sleep session. In other examples, the time series is representative of a sleep event probability over time, such as a sleep disordered breathing (SDB) event probability over time. In some examples, the time series may express, for each sample, the probability that an SDB event (e.g. an obstructive or central apnea, or a hypopnea) occurred at that time. Other examples of sleep events include cortical or autonomic arousals, periodic limb movements, or any other cortical or autonomic event occurring during, and associated with (normal, or disordered) sleep.

In some approaches, each set of training data comprises a different type of physiological signal. By combining the initial scores from score-based neural networks independently trained on different types of physiological signals, the generative model may be easily adapted for different combinations of physiological signals. This allows the generative model to be used regardless of the particular physiological signals available in a given case.

Each set of training data may comprise data from a different group of subjects. In other words, the processing system may use a federated learning technique.

The different groups of subjects may be grouped according to a clinical setting associated with each subject. This allows the generative model to make use of information from a large number of clinics, without requiring the clinics to share confidential patient data. Further, clinics can be added to or removed from the generative model without the need for retraining the entire model.

The different groups of subjects may be grouped according to one or more demographic criteria. For instance, subjects may be grouped according to one or more of age, comorbidities, severity of sleep disordered breathing, etc. The generative model may then be tailored to a subject being analyzed, by only using the initial scores from score-based neural networks trained for demographic groups to which the subject belongs.

In some examples, each set of training data comprises data from a different subject. This allows individual subjects to opt in or out of being used by the generative model, without requiring the entire generative model to be retrained.

The processing system may be further configured to, for each initial score: generate a plurality of samples by iteratively performing a sampling on the initial score; and process the plurality of samples to generate a measure of uncertainty of the initial score. The uncertainty may be any measure of statistical dispersion, such as a variance, standard deviation, interquartile range and so on.

The processing system may be configured to process the initial scores to generate a combined score by performing a process comprising combining only those initial scores whose measure of uncertainty meets one or more predetermined conditions.

In some examples, the processing system may be configured to generate a combined measure of uncertainty by combining the measure of uncertainty generated for each initial score. The combined measure of uncertainty may be output (e.g., displayed) alongside the output data of the generative model to indicate a measure of general (aleatoric) uncertainty of the overall generative model.

In an alternative approach, the combined measure of uncertainty is generated using the combined score. In particular, the method may comprise generating a plurality of second samples by iteratively performing a sampling on the combined score; and process the plurality of second samples to generate the combined measure of uncertainty.

There is also proposed a computer-implemented method, comprising: receiving a plurality of initial scores, wherein each initial score is an output of a different score-based neural network of a generative model and defines a probability distribution for sampling output data, wherein each score-based neural network has been independently trained for a different set of training data; processing the initial scores to generate a combined score; and processing the combined score, using a sampling technique, to produce output data of the generative model.

The computer-implemented method may be configured to perform the function of any herein disclosed processing system, and vice versa.

There is also proposed a computer program product comprising computer program code means which, when executed on a computing device having a processing system, cause the processing system to perform all of the steps of any herein disclosed method.

There is also proposed a respiratory support system for providing an airflow to a subject. The respiratory support system comprises a processing system, at least two sensors and an airflow control system. The processing system is as previously described, and configured to generate (as the output data) an output signal responsive to a predicted occurrence of respiratory difficulty of the subject. Each sensor is adapted to generate a different physiological signal of the subject. Each score-based neural network used by the processing system is configured to generate an initial score by processing a respective, different physiological signal. Thus, each score-based neural network is trained using a respective instance of training data for a same type of data as a respective one of the physiological signals. The processing system is configured to process the initial scores to generate a combined score; and process the combined score, using a sampling technique, to produce the output data of the generative model. The airflow control system is configured to control a property of the airflow to the subject based on the output data of the generative model, e.g., based on the predicted occurrence of any respiratory difficulty of the subject.

This approach provides a mechanism for active management of the airflow to a subject using directly monitored physiological signals. In particular, each physiological signal is independently processed using a separate or dedicated score-based neural network to produce a respective initial score. The initial scores are then combined, with the combined score being used to predict or generate an output signal responsive to a predicted occurrence of respiratory difficulty. This output signal is then used to control an airflow to the subject. Occasionally, a subject removes a sensor while using the respiratory support system, for example unintentionally while moving during sleep, or intentionally when feeling discomfort while trying to fall asleep. The processing system is able to generate the combined score based on the initial scores that are available, without the need for dedicated training for each specific combination of sensors. In another case, the subject replaces a sensor with another sensor. For example, the subject has been using a face mask with a sensor for a certain time. Due to wear of the face mask, the subject replaces the worn face mask with a face mask from a different brand or different model. The processing device is able to generate a new initial score based on the physiological signal from the sensor in the new face mask.

There is also proposed a sleep stage determination system for determining sleep stages of a subject. The sleep stage determination system comprises a processing system and at least two sensors. Each sensor is adapted to generate a different physiological signal of the subject. The processing system is embodied as previously described, wherein each score-based neural network is configured to generate an initial score by processing a different one of the physiological signals produced by the at least two sensors. Thus, each score-based neural network is trained using a respective instance of training data for a same type of data as a respective one of the physiological signals. The processing system is configured to process the initial scores to generate a combined score; and process the combined score, using a sampling technique, to produce the output data of the generative model. The output data is representative of one or more sleep stages of the subject.

These and other aspects of the invention will be apparent from and elucidated with reference to the embodiment(s) described hereinafter.

## BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a workflow for a proposed approach;
Fig. 2 is a flowchart illustrating a proposed method;
Fig. 3 is a flowchart illustrating a variation to the proposed method; and
Fig. 4 is a flowchart illustrating yet another proposed method.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating output data using a generative model. The generative model comprises a plurality of score-based neural networks, each configured to generate an initial score processable, using a sampling technique, to produce an instance of example data. The initial scores are combined to define a combined score. The combined score is processed using the sampling technique to generate the output data. Each score-based neural network is trained using a different set of training data.

The present disclosure provides a concept of using multiple score-based neural networks to produce separate scores, which are then combined to define the score from which output data is generated.

In some examples, different score-based neural networks may be trained to process input data of different sets of input (data) types. In effect, this means that if a certain input (data) type is unavailable, then the score-based neural network for that type can be deactivated, with the remaining score-based neural networks producing initial scores for combination. This increases a flexibility of use of the proposed generative model.

In the context of the present disclosure, an input (data) type represents input data for a particular variable. Thus, instances of input data of different input (data) types are instances of input data of different variables. The term input (data) type (i.e., input type or input datatype) and variable are therefore considered to be semantically interchangeable. Thus, different score-based neural networks may be trained to process input data of different sets or combinations of one or more input variables.

In some examples, different score-based neural networks may be trained using training data for different populations or groups of one or more subject. This effectively introduces a federated learning component to the generative model, improving the accuracy of the generative model by reducing a specificity to a particular population or group of subject (e.g., reducing unconscious bias of the generative model).

The present disclosure thereby effectively defines or provides a new architecture for a generative model.

In general, a score-based generative model receives some input data, which is processed in order to produce a prediction of some output data. One characteristic of a score-based generative model (also known as a diffusion model) is the use of a score derived from the input data, which defines or characterizes a probability distribution. A sampling technique is used to process the score (particularly the probability distribution) to generate or predict one or more values for the output data. In other words, the input data is used to define probable values for output data (e.g., in the form of a score for a probability distribution). The actual output data is produced by sampling the probable values using a sampling technique to guess or estimate the output data.

The present invention proposes a concept of generating multiple initial scores using different score-based neural networks (i.e., separate or individual diffusion models). Each score-based neural network is trained using different sets of training data. The different sets of training data may, for instance, represent training data produced for different populations and/or for different types/variables of input data. The multiple initial scores are then combined or fused to produce a combined score, which is used to define or derive the output data.

Some notation is hereafter defined to aid in the contextual understanding of the proposed approach. Nonetheless, it is noted that the general principle of a score-based neural network for generating a score for use in a generative model (specifically a diffusion model) is well known in the art, e.g., as set out by Karras, Tero, et al. "Elucidating the design space of diffusion-based generative models." Advances in Neural Information Processing Systems 35 (2022): 26565-26577 Song, Yang, et al. "Score-based generative modeling through stochastic differential equations." arXiv preprint arXiv:2011.13456 (2020).

The notation $x_i$ denotes an input signal or channel with index i, i.e., represents an instance of input data. The overall input data for a generative model may be formed from a plurality of input signals, e.g., up to N input signals. Each input signal may, for instance, represent a different variable, i.e., a different type or category of input data. Alternatively, different input signals may represent different subsets of input data. In some examples, one or more of the input signals are the same.

The notation y denotes output data, such as an output signal.

As a working example, in the context of sleep stage prediction, an input signal $x_i$ may represent any signal responsive to a sleep stage of a subject during a sleep session, such as an EEG signal; an ECG signal; a PPG signal; an audio signal (e.g., measured by a microphone); and/or a motion signal (e.g., responsive to a breathing motion of the subject). In this working example, the output data y may be a hypnogram depicting predicted sleep stages for the subject during the sleep session.

As another working example, in the context of sleep disordered breathing prediction, an input signal $x_i$ may represent any signal responsive to a disordered breathing of the subject during a sleep session, such as an EEG signal; an ECG signal; a PPG signal; an audio signal (e.g., measured by a microphone); and/or a motion signal (e.g., responsive to a breathing motion of the subject). In this working example, the output data y may be an output signal depicting the probability of an SDB event occurring over time.

As previously mentioned, a score represents or defines a probability distribution from which output data is derived, e.g., by sampling the probability distribution. In a working example, $\nabla_y$ ('del' or 'nabla') denotes the vector derivative of a function with respect to **y**. $\nabla_y \log p(\mathbf{y})$ and $\nabla_y \log p(\mathbf{y}|x_i)$ can represent different types of scores, in this case a prior score (e.g., a baseline score) and an initial score, respectively. That is, these scores are the vector derivates with respect to **y** of the logarithm of a probability distribution of **y**.

One aim of the proposed disclosure is to calculate a combined score $\nabla_y \log p(\mathbf{y}|\mathbf{x}_1,\mathbf{x}_2,...,\mathbf{x}_N)$ from which the output data can be generated. The combined score is produced by combining two or more initial scores and, optionally/preferably, the prior score. This combined score $\nabla_y \log p(\mathbf{y}|\mathbf{x}_1,\mathbf{x}_2,...,\mathbf{x}_N)$ effectively takes into account multiple input signals or subsets of input data in producing the combined score. This effectively produces a score given all measurement modalities available or present.

The combined score may effectively be a vector differential operator that defines or characterizes the probability distribution from which a predicted output can be derived.

In practice, it is functionally impossible to capture or express the prior score $\nabla_y \log p(\mathbf{y})$ and/or the initial scores $\nabla_y \log p(\mathbf{y}|\mathbf{x}_i)$ exactly. Instead, the present disclosure proposes that these scores are learned/approximated through application of neural networks. These neural networks can be any function parameterized by learnable parameters $\theta$ that take $\mathbf{y}$ and (for producing an initial score) $x_i$ as input and output a score. In equations this can be expressed as:

$$\nabla_y \log p(\mathbf{y}) \approx s_{\theta_0}(\mathbf{y}, \mathbf{0}) \tag{1}$$

$$\nabla_y \log p(\mathbf{y}|\mathbf{x}_i) \approx s_{\theta_j}(\mathbf{y}, \mathbf{x}_j) \tag{2}$$

Where s(.) Denotes the neural network. Each initial score is produced by a differently trained neural network. In particular, each neural network is trained using a different set of training data. Thus, the parameters $\theta$ for different neural networks are learned using different sets of training data. In some examples, the parameters may be learned per signal source (e.g., the index j may be equal to the index i). Moreover, the prior score can be viewed as a special case of the initial score, where i = 0 and $\mathbf{x}_0$=0.

There are several known types of neural network that are able to learn how to approximate scores, which are commonly referred to as score matching techniques. Known examples include sliced score matching and denoising score matching. The herein proposed invention is agnostic to the score matching technique used. Thus, the type of score matching technique employed is immaterial to proposed embodiments.

As previously mentioned, a goal of the proposed approach is to produce a combined score $\nabla_y \log p(\mathbf{y}|\mathbf{x}_1,\mathbf{x}_2, \dots, \mathbf{x}_N)$ by combining initial scores. One example technique for estimating the combined score from the initial scores (and optionally the prior score) is defined by the follow equation:

$$\nabla_y \log p(\mathbf{y}|\mathbf{x}_1, \mathbf{x}_2, \dots, \mathbf{x}_N) = \nabla_y \log p(\mathbf{y}) + \sum_{i=1}^{N} (\nabla_y \log p(\mathbf{y}|\mathbf{x}_i) - \nabla_y \log p(\mathbf{y})) \tag{3}$$

The scores on the right hand of the equation may be estimated by neural networks as in (1) and (2). In other words, the combined score is only ever produced or inferred during deployment, without needing training on a combined signal model.

It is of note that the gradient with respect to the source, $\nabla_y$, is taken, since the normalization constants disappear. As a consequence, it is a fundamental requirement to use score-based neural networks, instead of more classical deep learning approaches (e.g., cross entropy).

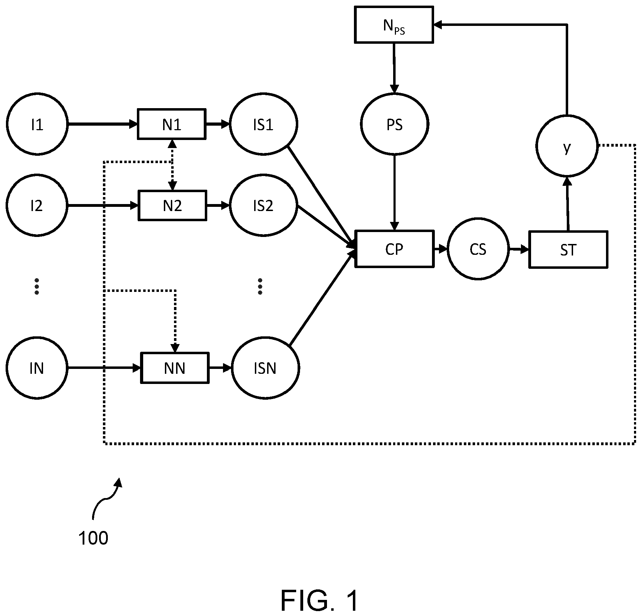

Fig. 1 conceptually illustrates an example workflow for a proposed approach.

In particular, Fig. 1 illustrates how initial scores can be combined to produce the combined score. In particular, a plurality of input signals I1, I2, ..., IN or input data portions are processed using a respective plurality of neural networks N1, N2, ..., NN to generate a respective plurality of initial scores IS1, IS2, ..., ISN.

Each neural network N1, N2, ... N3 may also process the output data y to generate the respective initial score. In this way, each neural network may effectively operate using the principle of denoising score matching, e.g., as explained or set out by Vincent, Pascal. "A connection between score matching and denoising autoencoders." Neural computation 23.7 (2011): 1661-1674; Karras, Tero, et al. "Elucidating the design space of diffusion-based generative models." Advances in Neural Information Processing Systems 35 (2022): 26565-26577 amongst others.

The plurality of initial scores are combined (by a combining process CP) to produce the combined score CS.

The combined score CS is then processed using a sampling technique ST to produce output data y. In particular examples, the combined score may define a probability distribution (or distributions) for the output data y. The probability distribution(s) are appropriately sampled by the sampling technique to then produce the output data.

There are many possible sampling techniques able to perform this procedure including: Langevin dynamics, denoising diffusion probabilistic models (DDPM), denoising diffusion implicit models (DDIM), and ordinary differential equation (ODE) solvers such as Euler's method or higher order Runge-Kutta methods. The proposed approach is agnostic to the precise sampling used. Thus, the type of sampling technique employed is immaterial to proposed embodiments.

In some instances, a prior score PS is also generated by processing a previous prediction for the output data y using an appropriately trained neural network $N_{PS}$. The generation of the combined score CS may further use the prior score (as well as the initial scores) to produce the combined score, e.g., by employing the approach described by equation (3).

In this way, the production of the output data may be an iterative approach to iteratively update a predicted output y using input data I1, I2, ..., IN. Thus, the proposed approach may effectively aim to solve the question of "based on a current version of the output, what is a better estimate of that output given an input?".

It has previously been mentioned how a score-based neural network is used to process input data to produce an initial score. In particular, different score-based neural networks produce different initial scores, which are then combined in producing a combined score.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a neural network to perform the function of score generation are well known. As a general overview, such a neural network can be trained by adding noise to training data and training the neural network to denoise this training data to thereby define the (trained) score-based neural network.

Example approaches for suitably training a score-based neural network are described by Song, Yang, et al. "Score-based generative modeling through stochastic differential equations." arXiv preprint arXiv:2011.13456 (2020); Karras, Tero, et al. "Elucidating the design space of diffusion-based generative models." Advances in Neural Information Processing Systems 35 (2022): 26565-26577; Ho, Jonathan, Ajay Jain, and Pieter Abbeel. "Denoising diffusion probabilistic models." Advances in neural information processing systems 33 (2020): 6840-6851; and Pang, Tianyu, et al. "Efficient learning of generative models via finite-difference score matching." Advances in Neural Information Processing Systems 33 (2020): 19175-19188. This list of example approaches is non-exhaustive, and the skilled person will readily appreciate a wide variety of other approaches and/or techniques for suitably training a neural network to perform a score generating function.

As previously explained, different score-based neural networks are trained using different sets of training data. Thus, the training data for training a nth score-based neural network may comprise a nth set of training data.

The skilled person will appreciate that there is a direct connection between the training data used to train a particular score-based neural network and the input data processed by the neural network during later inference (to produce the initial score). In particular, the input data processed during inference contains the same type of data (e.g., representing the same or comparable (e.g., surrogate version of the) property/properties or variable(s)) as that of the example input data in the training data used to train the score-based neural network.

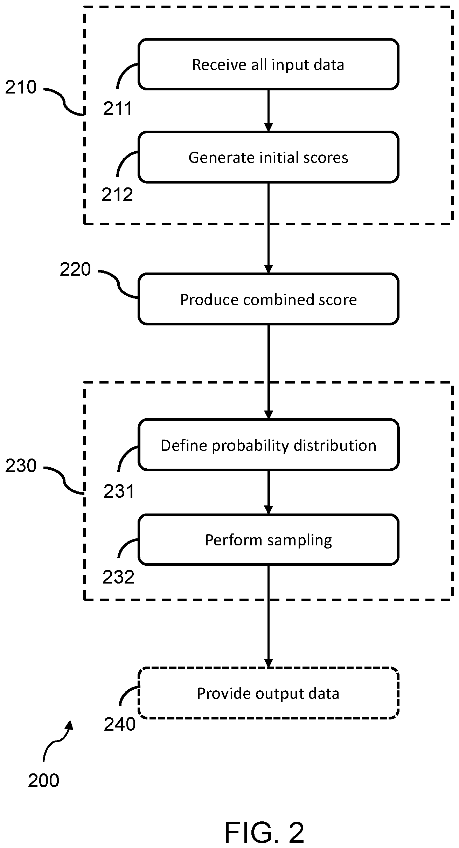

Fig. 2 is a flowchart illustrating a proposed method 200. The method 200 is a computer-implemented method, which may be executed by a processing system, suitable embodiments of which are later described. The method 200 describes an inference process that makes use of a generative model having a herein-proposed architecture, e.g., employs a plurality of different score-based neural networks.

The method comprises a step 210 of receiving a plurality of initial scores, wherein each initial score is an output of a different score-based neural network of a generative model and defines a probability distribution for sampling output data, wherein each score-based neural network has been independently trained for a different set of training data.

Step 210 may comprise, for instance, a sub-step 211 of receiving a plurality of instances of input data. Each instance of input data may, for example, represent an input signal generated by a different sensor or defined in a different field or entry of a data record for a subject.

Two or more sets of one or more instances of input data is then processed, in a sub-step 212, by a respective score-based neural network to generate an initial score. Thus, each score-based neural network processes one or more instances of input data to produce an initial score. Different score-based neural network process different sets of (one or more) instances of input data. As previously mentioned, each score-based neural network has been independently trained for and/or using a different set of training data, e.g., has been designed to process a particular set (e.g., combination) of one or more instances of input data. Suitable examples of instances of input data and/or sets of training data will be provided later in this disclosure.

The method 200 also comprises a step 220 of processing the initial scores to generate a combined score. Suitable approaches for performing step 220 have been previously described.

The method 200 also comprises a step 230 of processing the combined score, using a sampling technique, to produce output data of the generative model. In particular, step 230 may comprise a sub-step 231 of defining a probability distribution using the combined score. Step 230 may then perform a sub-step 232 of processing the probability distribution using a sampling technique to produce the output data. Examples of suitable sampling techniques have been previously described, and would be otherwise apparent to the person skilled in the art.

It has been previously described how different score-based neural networks are trained using different sets of training data.

In a first working example, each score-based neural network is trained to process input data of a different set of one or more input (data) types (representing one or more different variables) to produce a respective initial score. Thus, during a training process, one score-based neural network will be trained using one set of training data (containing example input data for a first set of input (data) types) and another score-based neural network will be trained using a different set of training data (containing example input data for a second, different set of input (data) types).

An input (data) type defines an expected source for a corresponding instance of input data. Thus, for instance, input data of a first type is produced or otherwise provided by a first source (e.g., a first sensor) and input data of a second type is produced or otherwise provided by a second, different source (e.g., a second sensor).

In this first working example, during inference using the trained score-based neural networks, each score-based neural network will process input data produced from a different set of input sources to generate initial score. This produces a different initial score for different sets or combinations of input sources, which are then combined to produce the combined score.

In a second working example, each score-based neural network is trained to process a same set of one or more input (data) types to produce a respective score. However, during the training process, different training datasets are used to train different neural networks.

As one example, different training datasets may represent data from different populations of subjects (e.g., different demographics, locations and so on). Thus, each set of training data may comprise data from a different group of subjects. As a working example, each separate training dataset may belong to a specific demographic of subject, such as age brackets, presence of comorbidities, or severity of sleep disordered breathing.

As another example, different training datasets may represent data from a different single subject or individual. Thus, each set of training data may comprise data from a different subject. Thus, it is effectively possible to aggregate the score-based neural networks built for different subjects/individuals. Individual subjects can readily opt-in and opt-out at any time for their score-based neural network to be used, without the need for retraining the entire algorithm. Rather, the individual score-based neural network can be simply left out of combination process.

In this second working example, during inference using the trained score-based neural network, each score-based neural network will process input data produced from a same set of input sources to generate initial scores. As each score-based neural network has been trained using different datasets, then it will be apparent that different initial scores will be produced.

This second working example effectively provides a federated learning technique to generate the combined score.

Of course, a combination of these two working examples may also be performed. For instance, each score based neural network may be trained using datasets for different populations of subjects and/or to process a different set of one or more input (data) types. Thus, in some examples, there may be at least three score-based neural networks of which at least two of the score-based neural networks may be trained using datasets for different populations of subjects and at least two of the score-based neural networks may be trained to process different sets of input (data) types. As another example, there may be a plurality of score-based neural networks for which one score-based neural network is trained using a first populations of subject and to process a first set of input (data) types, with another score-based neural network being trained using a second (different) population of subjects and a second (different) set of input (data) types.

The method 200 may further comprise a step 240 of outputting or otherwise providing the output data (generated in step 230). Step 240 may, for instance, comprise controlling a user interface to provide a visual representation of the output data. In examples, step 240 may comprise storing the output data in a memory or storage system. Step 240 may also/otherwise comprise processing the output data (e.g., using a further algorithmic process). Step 240 may be carried out by a separate processing system, for examples. Step 240 may comprise performing any combination of one or more of the above-described examples.

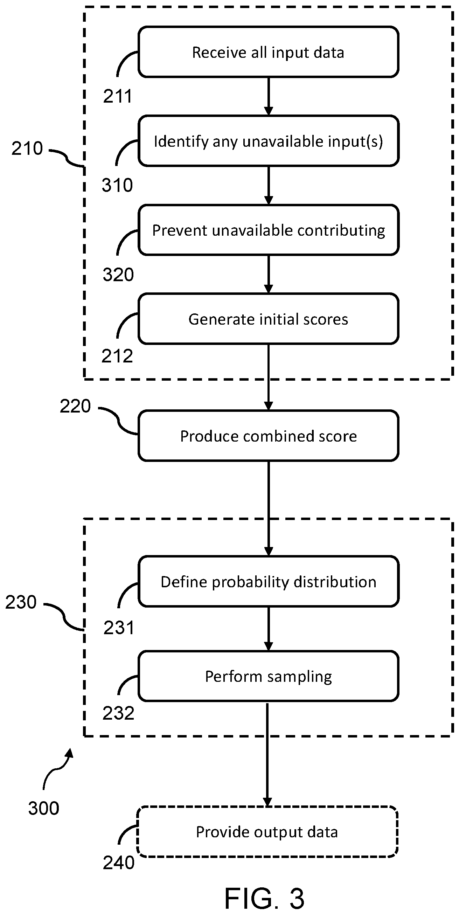

Fig. 3 is a flowchart illustrating a method 300, which is a variation of the previously described method 200 described with reference to Fig. 2. In this variation, it is assumed that at least two of the score-based neural networks are configured to process a different set of one or more input (data) types.

The method 300 comprises a modified version of step 210. In particular, step 210 further comprises a step 310 of identifying one or more available types of input data for determining the output data. Step 210 further comprises a step 320 of identifying any of the score-based neural network that requires an unavailable type of input data to produce an initial score. Responsive to a positive determination/identification in step 320, step 210 performs a step 330 of preventing any identified score-based neural network, in step 320, from contributing to the combined score. In particular, step 330 may comprise preventing any identified score-based neural network, in step 320, from producing an initial score.

Of course, step 220 may appropriately comprise preventing any initial score produced by a score-based neural network that requires an unavailable type of input data from contributing to the combined score.

This approach can effectively adjust, on the fly (e.g., per subject), the generation of the combined score based on the availability of input data for producing the initial score. This advantageously provides a generative model architecture that is adaptive to the circumstances in which it is deployed.

In the context of the present disclosure, a type of input data may be considered to be unavailable if the corresponding input data is not obtained or is obtained at an insufficiently high quality. Approaches for determining a quality of input data are well known in the art, including approaches that monitor a signal-to-noise ration of the input data. This advantageously avoids the need to repeat a sampling or recording of input data if the production of a particular type of input fails, for instance.

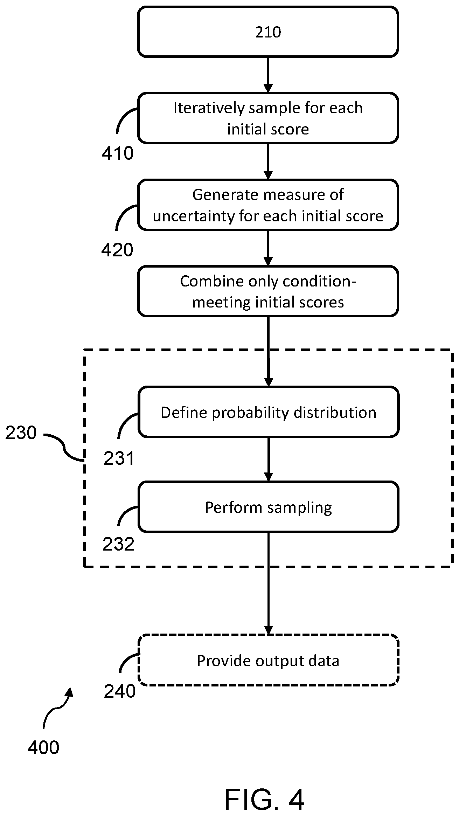

Fig. 4 is a flowchart illustrating a method 400 that provides additional optional steps or processes for any previously described method 200, 300 for generating an output for a generative model.

The method 400 is configured to only allow initial scores that meet one or more predetermined quality conditions to contribute to the combined score. In particular, the method 400 is configured such that only those initial scores that are able to produce output data with a relatively high certainty are permitted to contribute to the combined score.

The method 400 further comprises performing a step 410 of, for each initial score, generating a plurality of samples by iteratively performing a sampling on the initial score. Any suitable sampling technique may be employed in step 410. Preferably, the same sampling technique as used in step 230 is employed in step 410 to same each initial score in producing the plurality of samples.

The method 400 further comprises a step 420 of processing the plurality of samples to generate a measure of uncertainty of the initial score. Any known measure of uncertainty may be determined in step 420, examples of which include any measure of statistical dispersion, such as a variance, standard deviation, interquartile range and so on.

The step 220 may be adapted to comprise combining only those initial scores whose measure of uncertainty meets one or more predetermined conditions. Thus, with reference to Fig. 1, only those initial scores that meet the one or more predetermined conditions may form part of the initial scores IS1, IS2, ..., ISN that are processed to produce the combined score.

Examples of suitable predetermined conditions include a measure of uncertainty being below a predetermined value, e.g., a standard deviation being below a predetermined value. Other example conditions will be readily apparent to the suitably skilled person in the art.

Proposed approaches can be employed or applied in a wide variety of circumstances and/or situations. A number of working examples are hereafter described.

In one working example, the proposed approach is employed for the classification of sleep stages during a sleep session of a subject.

The input data to be processed by the score-based neural networks (to produce the initial score(s)) may comprise two or more sets of one or more physiological signals known to correlate or be otherwise responsive to a sleep stage of the subject. Suitable examples include neurological signals, such as EEG, EOG, EMG, but can also include alternative sensor modalities, which characterize sleep from an autonomic nervous system activity perspective, such as cardiac signals (e.g., ECG, PPG, BCG, SCG), or respiratory signals (such as those measured with thoracic and/or abdominal respiratory belts, bed sensors, Doppler radars, chest-mounted accelerometers, etc.).

Each score-based neural network may be configured (i.e., be appropriately trained) to process a different set of one or more physiological signals in order to produce a respective initial score. Thus, each set of physiological signals may be processed by a different score-based neural network to produce a plurality of initial scores. The initial scores are then combined, using previously described approaches, to produce a combined score. The combined score is then processed, using a sampling algorithm and previously described approaches, to predict a time series of data representing predicted sleep stages of the subject through the sleep session.

In another working example, the proposed approach is employed for the detection of sleep-disordered breathing (SDB).

The input data to be processed by the score-based neural networks (to produce the initial score(s)) may comprise two or more sets of one or more physiological signals known to correlate or be otherwise responsive to sleep-disordered breathing. Examples include those same physiological signals set out above for the sleep-stage prediction example. Further examples include physiological signals produced by sensors that measure the direct effects of SDB events, such as airflow sensors (e.g., nasal pressure cannulas, or oronasal thermistors), or oxygen saturation sensors (e.g., finger-mounted transmissive SpO2, or transmissive SpO2 mounted on other parts of the body).

As before, each score-based neural network may be configured (i.e., be appropriately trained) to process a different set of one or more physiological signals in order to produce a respective initial score. Thus, each set of physiological signals may be processed by a different score-based neural network to produce a plurality of initial scores. The initial scores are then combined, using previously described approaches, to produce a combined score. The combined score is then processed, using a sampling algorithm and previously described approaches, to predict a time series of data representing predicted occurrences (or non-occurrence) of sleep disordered breathing of the subject through the sleep session.

The output data of the generative model may be used to control the operation of a respiratory support device for the subject, e.g., to control a property of an airflow provided to a subject. This facilitates direct control over the operation of a respiratory support device based on the predicted occurrence of sleep disordered breathing, for improved control over the mechanically-aided breathing of the subject.

Thus, there is provided a respiratory support device for providing an airflow to a subject. The respiratory support device comprises a processing system configured to carry out a previously described method for generating an output using a generative model. The respiratory support device also comprises at least two sensors, each adapted to generate a physiological signal of the subject. Each score-based neural network is trained for processing a different set of the physiological signals to produce a respective initial score. Thus, each score-based neural network has been independently trained using a different set of training data, wherein the different sets of training data contain data for a same type or property as each set of physiological signals. The processing system is configured generate an initial score for each set of physiological signals produced by the at least two sensors and control a property of the airflow based on the output data produced by the generative model. Examples of suitable properties include pressure, pressure profile, flowrate, temperature, and/or humidity.

In another working example, the proposed approach is employed for the segmentation of an anatomical element, such as a tumor or growth, in image data.

In this working example, the input data to be processed by the score-based neural networks (to produce the initial score(s)) may comprise two or more instances of image data, e.g., of different imaging modalities, of a same region of interest. Examples include MRI images, CT images, PET images, ultrasound images and so on.

Each score-based neural network may be configured (i.e., be appropriately trained) to process a different modality of image in order to produce a respective initial score. Thus, each instance of image data in the input data may be processed by a different score-based neural network to produce a plurality of initial scores. The initial scores are then combined, using previously described approaches, to produce a combined score. The combined score is then processed, using a sampling algorithm and previously described approaches, to predict a segmentation of the anatomical element in at least one of the instances of image data. Thus, the output data of the generative model is a segmentation of the anatomical element.

The proposed approach or technique can be applied to perform a wide variety of technical tasks. Proposed approaches are particularly advantageous when used to process or analyze medical data in order to perform a medical analysis task.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1.   A processing system configured to:

   receive a plurality of initial scores, wherein each initial score is an output of a different score-based neural network of a generative model and defines a probability distribution for sampling output data, wherein each score-based neural network has been independently trained using a different set of training data;
   process the initial scores to generate a combined score; and
   process the combined score, using a sampling technique, to produce output data of the generative model.

2.   The processing system of claim 1, wherein each initial score is a vector differential operator.

3.   The processing system of claim 1 or 2, wherein:

   each set of training data comprises training data for a respective one of a plurality of different data types; and
   the processing system is further configured to:

      receive a plurality of sets of input data, each set of input data containing input data for a respective one of the plurality of different data types; and
      provide each set of input data to a score-based neural network trained using a set of training data of the same type to generate the plurality of initial scores.

4.   The processing system of any of claims 1 to 3, wherein the generative model is a clinical assessment tool.

5.   The processing system of claim 4, wherein the output data of the generative model is a time series.

6.   The processing system of claim 5, wherein the sets of training data together comprise physiological signals for one or more subjects during one or more sleep sessions.

7.   The processing system of claim 6, wherein the physiological signals comprise one or more of: an electroencephalography signal, an electrooculography signal, an electromyography signal, an electrocardiography signal, a ballistocardiography signal, a seismocardiography signal, a pulse oximetry signal, and/or a respiratory signal.

8.   The processing system of claim 6 or 7, wherein the time series is a hypnogram or is representative of an SDB event probability over time.

9.   The processing system of any of claims 6 to 8, wherein each set of training data comprises a different type of physiological signal.

10.   The processing system of any of claims 6 to 9, wherein each set of training data comprises data from a different group of subjects.

11.   The processing system of any of claims 6 to 10, wherein each set of training data comprises data from a different subject.

12.   The processing system of any of claims 1 to 11 further configured to, for each initial score:

generate a plurality of samples by iteratively performing a sampling on the initial score; and
process the plurality of samples to generate a measure of uncertainty of the initial score.

13. The processing system of claim 12, wherein the processing system is configured to process the initial scores to generate a combined score by performing a process comprising combining only those initial scores whose measure of uncertainty meets one or more predetermined conditions.

14. A computer-implemented method, comprising:

receiving a plurality of initial scores, wherein each initial score is an output of a different score-based neural network of a generative model and defines a probability distribution for sampling output data, wherein each score-based neural network has been independently trained for a different set of training data;
processing the initial scores to generate a combined score; and
processing the combined score, using a sampling technique, to produce output data of the generative model.

15. A computer program product comprising computer program code means which, when executed on a computing device having a processing system, cause the processing system to perform all of the steps of the method according to claim 14.

FIG. 1

FIG. 2

FIG. 3

FIG. 4

Europäisches
Patentamt

European
Patent Office

Office européen
des brevets

# EUROPEAN SEARCH REPORT

Application Number

EP 23 19 4855

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/230276 A1 (CLARK AIDAN [GB] ET AL) 21 July 2022 (2022-07-21) * paragraphs [0044], [0086] - [0089]; figures 1,2,3 * | 1-15 | INV. G16H50/20 |
| A | YANG SONG ET AL: "Improved Techniques for Training Score-Based Generative Models", ARXIV.ORG, CORNELL UNIVERSITY LIBRARY, 201 OLIN LIBRARY CORNELL UNIVERSITY ITHACA, NY 14853, 16 June 2020 (2020-06-16), XP081697077, * the whole document * | 1-15 | |
| A | LI JIN ET AL: "Wavelet Transform-Assisted Adaptive Generative Modeling for Colorization", IEEE TRANSACTIONS ON MULTIMEDIA, IEEE, USA, vol. 25, 25 May 2022 (2022-05-25), pages 4547-4562, XP011951664, ISSN: 1520-9210, DOI: 10.1109/TMM.2022.3177933 [retrieved on 2022-05-26] * the whole document * | 1-15 | |
| A | US 2022/398697 A1 (VAHDAT ARASH [US] ET AL) 15 December 2022 (2022-12-15) * figure 2 * | 1-15 | TECHNICAL FIELDS SEARCHED (IPC) G16H |

The present search report has been drawn up for all claims

| Place of search | Date of completion of the search | Examiner |
|---|---|---|
| Munich | 2 February 2024 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 4 517 774 A1

## ANNEX TO THE EUROPEAN SEARCH REPORT
## ON EUROPEAN PATENT APPLICATION NO.

EP 23 19 4855

02-02-2024

| Patent document cited in search report | | Publication date | Patent family member(s) | | Publication date |
|---|---|---|---|---|---|
| US 2022230276 | A1 | 21-07-2022 | EP | 3973459 A1 | 30-03-2022 |
| | | | US | 2022230276 A1 | 21-07-2022 |
| | | | WO | 2020234449 A1 | 26-11-2020 |
| US 2022398697 | A1 | 15-12-2022 | CN | 115526223 A | 27-12-2022 |
| | | | DE | 102022113243 A1 | 08-12-2022 |
| | | | US | 2022398697 A1 | 15-12-2022 |

# EP 4 517 774 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **KARRAS, TERO et al.** Elucidating the design space of diffusion-based generative models. *Advances in Neural Information Processing Systems*, 2022, vol. 35, 26565-26577
- **SONG, YANG et al.** Score-based generative modeling through stochastic differential equations. *arXiv:2011.13456*, 2020
- **VINCENT, PASCAL**. A connection between score matching and denoising autoencoders. *Neural computation*, 2011, vol. 23 (7), 1661-1674
- **KARRAS, TERO et al.** Elucidating the design space of diffusion-based generative models.. *Advances in Neural Information Processing Systems*, 2022, vol. 35, 26565-26577

- **SONG, YANG et al.** Score-based generative modeling through stochastic differential equations.. *arXiv:2011.13456*, 2020
- **HO, JONATHAN ; AJAY JAIN ; PIETER ABBEEL**. Denoising diffusion probabilistic models. *Advances in neural information processing systems*, 2020, vol. 33, 6840-6851
- **PANG, TIANYU et al.** Efficient learning of generative models via finite-difference score matching. *Advances in Neural Information Processing Systems*, 2020, vol. 33, 19175-19188